(19) Europäisches Patentamt / European Patent Office / Office européen des brevets

(11) EP 4 520 837 A1

(12) EUROPEAN PATENT APPLICATION

(43) Date of publication:
**12.03.2025 Bulletin 2025/11**

(21) Application number: **23306475.7**

(22) Date of filing: **05.09.2023**

(51) International Patent Classification (IPC):
***C12P 7/62*** *(2022.01)* ***C12N 9/18*** *(2006.01)*
***C12N 9/50*** *(2006.01)*

(52) Cooperative Patent Classification (CPC):
**C12P 13/001; C08G 59/302; C12P 7/62; C12Y 301/01001; C12Y 301/01003; C12Y 301/01013; C12Y 304/21; C12Y 304/21062;** C12N 9/18; C12N 9/50

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Essilor International**
**94220 Charenton-le-Pont (FR)**

(72) Inventors:
- **ZAPARUCHA, Anne**
  **91440 Bures-Sur-Yvette (FR)**
- **JADHAV, Dipesh**
  **31400 Toulouse (FR)**

(74) Representative: **Santarelli**
**Tour Trinity**
**1 bis Esplanade de la Défense**
**92035 Paris La Défense Cedex (FR)**

Remarks:
The complete document including Reference Table(s) and the Sequence Listing(s) can be downloaded from the EPO website

# (54) METHOD AND KIT FOR THE ENZYMATIC DEGRADATION OF POLYTHIOURETHANES

(57) The present disclosure relates to a method for the enzymatic degradation of polythiourethanes, consisting in contacting at least one enzyme of the invention with a polythiourethane in a suitable form, for a sufficient time and in nontoxic and environmentally friendly reaction conditions.

The method can also be used to recover the basic monomer and/or other compounds derived from the enzymatic degradation process, which can be further used to re-obtain the polymer or as intermediates in the synthesis of other chemicals.

The present disclosure also covers a kit for the enzymatic degradation of polythiourethanes.

EP 4 520 837 A1

## Description

### FIELD OF THE INVENTION

The present Invention belongs to the domain of polymer recycling, and more specifically, of polymer recycling via enzymatic degradation.

Indeed, the present Invention concerns a method for the enzymatic degradation of polythiourethanes (PTURs).

The Invention discloses a method and kit using enzymes capable of degrading PTURs in non-toxic and environmentally friendly conditions.

By degrading PTURs, the method of the invention allows reducing polymer waste as well as recovering the basic monomer and/or other chemicals, which can be further used to re-obtain the original PTURs or as source materials or intermediates in the synthesis of other chemicals.

### BACKGROUND OF THE INVENTION

Plastics are synthetic polymers that are ubiquitous ever since their production began from the year 1950's. Starting from this time, plastics have been produced at an increased rate to meet the demand of the entire world.

Environmental pollution by plastic waste started to be reported already in the 1970s and, by now, accumulation of plastic wastes in the environment is a major global problem because these materials are recalcitrant to natural biodegradation processes.

As a response to this problem, increasing efforts to reduce the plastic waste by disposing off plastics through segregated collection and recycling are underway. Despite these efforts, however, of all the plastic that is produced worldwide, only a minor fraction is at present successfully tackled with. The rest goes into the environment, either disposed of by landfilling, incinerated or in the oceans leading to water pollution. All these waste disposal strategies contribute to environmental pollution.

Even if plastics in the environment are degraded by photo-, bio-, and thermo-oxidative depolymerization as well as by friction, such biodegradation can take long periods of time, ranging from 50 years to more than 100 years.

As a consequence, scientists started to investigate ways of dealing with the vast accumulation of plastics that are more efficient and faster with respect to the above mentioned strategies of plastic waste disposal.

It has been found that a number of microorganisms are capable of producing enzymes that can degrade certain polymers (Tsushima *et al.,* 2010). The enzymes that are known to degrade plastic polymers belong to the class of "hydrolases". Enzymes belonging to this class are involved in a catalytic reaction which causes the breakdown of chemical bonds of their substrates in the presence of water. The hydrolases capable of degrading certain plastics are capable of hydrolyzing one or more chemical bonds within many of the commonly used plastics. The enzymes so far identified include esterases, lipases, PETases, laccases, polyurethanases and cutinases. The enzymatic degradation of plastic polymers results in simpler fragments, oligomeric or monomeric units. Microorganisms can further assimilate these smaller units into the microbial cell for further enzymatic degradation and release of metabolic products such as $CO_2$, $H_2O$, $CH_4$, and $N_2$ (Amobonye *et al.,* 2021). Many of these enzymes have been isolated from algae, actinomycetes, bacteria and fungi (Urbanek *et al.,* 2020).

Extraction of these enzymes and their modification in order to increase their enzymatic activity has been one of the main research areas in order to cope with ever increasing plastic pollution. Many types of plastics including polyethylene (PE), polyethylene terephthalate (PET), polylactic acid (PLA), polybutylene succinate (PBS) and polyurethane (PU) have been reported to undergo slow degradation in presence of these enzymes (Mukai *et al.,* 1993).

The use of such enzymes not only allows reducing the problem of increasing plastic pollution. In fact, the metabolic by-products of plastic degradation, some of which certainly harmful, can be recovered and re-cycled to re-obtain the original polymers and/or to synthesize other chemicals. This circular economy model allows reducing the environmental impact of plastics, thereby increasing the sustainability of their production.

Polythiourethanes (PTURs) are very versatile polymers due to their excellent physical, mechanical and optical properties.

PTURs may be synthesized, for example, by reacting a polythiol compound with a polyisocyanate or a polyisothiocyanate compound.

The versatility of PTURs derives from the possibility to alter their physical properties through the modulation of the physical properties of the polythiol compounds and/or of the polyisocyanate or polyisothiocyanate compounds.

The main application of this class of polymers is the production of optical materials thanks to their excellent optical characteristics and mechanical properties. In comparison to polyurethanes polymers (PUs), the incorporation of sulfur atoms into the polymeric chain increases flexibility, crystallinity and other useful properties. Moreover, the enhanced refractive index makes PTURs more suitable for optical applications.

As with most polymers, PTURs are primarily produced from non-renewable resources, which is problematic

considering their large production volumes.

A way to increase the sustainability of PTURs production is to move from the current linear PTURs production model to a more circular one. A circular model would minimize the negative impact of PTURs on the environment (i.e., would reduce its carbon footprint) and address the end-of-life issues of these polymers.

A circular economy approach can be realized by the development of methods for the biological depolymerization of PTURs. The idea behind biological depolymerization is to obtain raw materials (monomers, oligomers, polymer fragments and/or other chemicals) by energy-efficient and environmentally benign processes of polymer degradation, that can be used to re-obtain the original PTURs and/or for the synthesis of other polymers or chemicals.

Despite their extensive use and their industrial interest, no method has currently been reported for the enzymatic degradation of PTURs. Such a method would be of great interest because it would allow the re-cycling of the large quantities of PTURs generated by the industry, therefore contributing to reduce the environmental impact of its production and use.

## DISCLOSURE OF THE INVENTION

As described in the experimental part below, the inventors discovered that certain enzymes are capable of degrading polythiourethanes.

In light of these findings, the present invention aims to remedy to some or all of the above-mentioned problems of the prior art by proposing a method for the enzymatic degradation of polythiourethanes.

It should be noted that, at the time of filing of the present Application, no enzyme and method for the enzymatic degradation of polythiourethanes have been disclosed.

As used herein, the terms "comprise", or variations such as "comprises" or "comprising" as well as "including" and variations such as "include", "includes," and "included", are not limiting.

The term "about", when used before a numerical value, indicates that the value may vary within reasonable range, such as $\pm$ 10%, $\pm$ 5%, and $\pm$ 1%. The expression "about x" includes the value "x."

As used herein, the term "polymer" refers to a chemical compound which comprises repeating units linked by covalent chemical bonds.

As used herein, the term "repeating unit" is a part of a polymer whose repetition produces the complete polymer chain (except for the end-groups) by linking the repeat units together along the chain.

The repeating unit is not to be confused with the term "monomer", which refers to the small molecule from which a polymer is synthesized. In this sense, for example, repeating units can also be made from two or more monomers.

As used herein, the term "oligomer" refers to the polymer of the invention consisting of 2 to 50, preferably 2 to 20, repeating units.

The polymer according to the invention is a polythiourethane. As used herein, the term "polythiourethane" refers to a polymer containing the thiourethane bond of formula -S-C(=O)-N(H)- in every repeating unit of the polymeric chain.

Polythiourethanes are also known in the literature under the name of polythiocarbamates.

The polythiourethane as implemented in the present invention may be prepared by any known method, for example by reacting a polythiol compound with a polyisocyanate or a polyisothiocyanate compound.

The polythiol compound may be a typical one used in the synthesis of polythiourethanes.

More specifically, the polythiol compound is a compound of formula (I):

$$R^1(SH)_{n1} \qquad (I)$$

wherein n1 represents an integer ranging from 2 to 6 and $R^1$ represents an aliphatic, alicyclic, heterocyclic or aromatic group.

For example, the polythiol compound may comprise one or more compounds selected from the group consisting of: pentaerythritol tetrakis(3-mercaptopropionate), pentaerythritol tetrakis(2-mercaptoacetate), tris(3-mercaptopropionate) trimethylolpropane, tris(mercaptoacetate) trimethylolpropane, 2,3-bis((2-mercaptoethyl)thio)-1-propanethiol, ethylene glycol bis (3-mercaptopropionate), 2-mercaptoethyl 2-mercaptoacetate, 2-mercaptoethyl 3-mercaptopropionate, 5,7-dimercaptomethyl-1,11-dimercapto-3,6,9-trithiaundecane, 4,7-dimercaptomethyl-1,11 -dimercapto-3,6,9-trithiaundecane, 4-mercaptomethyl-1,8-dimercapto-3,6-dithiaoctane, 1,2-bis(2-mercaptoethylthio)-3-mercaptopropane, 2,5-bis (mercaptomethyl)-1,4-dithiane, bis (mercaptoethyl) sulfide, 1,1,3,3-tetrakis (mercaptomethylthio) propane, 4,6-bis (mercaptomethylthio)-1,3-dithiane, 2-(2,2-bis (mercaptomethylthio) ethyl)-1,3-dithietane, 1,1,2,2-tetrakis (mercaptomethylthio) ethane, 3-mercaptomethyl-1,5-dimercapto-2,4-dithiapentane, and tris (mercaptomethylthio) methane, dipentaerythritol hexakis(3-mercaptopropionate), tris[2-(3-mercaptopropionyloxy)ethyl] isocyanurate, butylene glycol dimercaptopropionate and ethanedithiol.

Preferably, the polythiol compound may be at least one selected from the group consisting of 1,2-bis(2-mercaptoethylthio)-3-mercaptopropane and pentaerythritol tetrakis (3-mercaptopropionate).

The polyisocyanate or polyisothiocyanate compound may be one typically used in the synthesis of polythiourethanes.

More specifically, the polyisocyanate or polyisothiocyanate is a compound of formula (II):

$$R^2(NCX)_{n2} \quad \text{(II)}$$

wherein X represents O or S, n2 represents an integer ranging from 2 to 6 and $R^2$ represents an aliphatic, alicyclic, heterocyclic or aromatic group.

For example, the polyisocyanate or polyisothiocyanate compound may comprise one or more compounds selected from the group consisting of toluene-2,4-diisocyanate, toluene-2,6-diisocyanate, diphenylmethane-4,4'-diisocyanate, diphenylmethane-2,4'-diisocyanate, paraphenylene diisocyanate, m-xylylene diisocyanate, biphenyl-diisocyanate, 3,3'-dimethyl-4,4'-diphenylene diisocyanate, tetramethylene-1,4-diisocyanate, hexamethylene-1,6-diisocyanate, 2,2,4-trimethyl hexane-1,6-diisocyanate, lysine methyl ester diisocyanate, bis(isocyanatoethyl) fumarate, isophorone diisocyanate, ethylene diisocyanate, dodecane-1,12-diisocyanate, cyclobutane-1,3-diisocyanate, cyclohexane-1,3-diisocyanate, cyclohexane-1,4-diisocyanate, hexahydrotoluene-2,4-diisocyanate, tetramethylxylylene diisocyanate, perhydro diphenylmethane-2,4'-diisocyanate, 4,4'-dicyclohexylmethanediisocyanate, bis(isocyanatomethyl)cyclohexane, dicyclohexylmethane diisocyanate, 2,5-bis(isocyanatomethyl)bicyclo-[2.2.1]-heptane and 2,6-bis(isocyanatomethyl)bicyclo-[2.2.1]-heptane, naphtalene diisocyanate, pentamethylene diisocyanate, HDI-trimer and PDI-trimer.

Preferably, the polyisocyanate or polyisothiocyanate compound may be at least one selected from the group consisting of m-xylylene diisocyanate and 2,5-bis(isocyanatomethyl)bicyclo[2.2.1]heptane.

According to a specific embodiment, the polythiourethane as implemented in the present invention is either MR-7 (refractive index of 1.67) or MR-8 (refractive index of 1.60).

MR-7 is a polythiourethane that can be synthesized starting from:

(i) m-xylylene diisocyanate, also known under the acronym "XDI" and
(ii) 1,2-bis(2-mercaptoethylthio)-3-mercaptopropane, also known under the alternative names of 4-mercaptomethyl-3,6-dithia-1,8-octanedithiol and 2,3-bis((2-mercaptoethyl)thio)-1-propanethiol.

MR-8 is a polythiourethane that can be synthesized starting from:

(i) 2,5-bis(isocyanatomethyl)bicyclo[2.2.1]heptane, also known under the acronym "NDI" or with the alternative names of norbornane-2,5-diylbis(methylene)diisocyanate, bicyclo[2.2.1]heptane,2,5-bis(isocyanatomethyl)- and 2,5-bis(isocyanatomethyl)bicyclo-[2.2.1]-heptane,
(ii) 1,2-bis(2-mercaptoethylthio)-3-mercaptopropane, also known under the alternative names of 4-mercaptomethyl-3,6-dithia-1,8-octanedithiol and 2,3-bis((2-mercaptoethyl)thio)-1-propanethiol, and
(iii) pentaerythritol tetrakis (3-mercaptopropionate).

Other polythiourethanes that can be enzymatically degraded according to the method of the invention are MR-6 and MR-10.

These optical materials as well as the monomers used for their preparation are especially described in the patents US 4,689,387, US 4,775,733, US 5,059,673, US 5,087,758 and US 5,191,055.

MR-6, MR-7, MR-8 and MR-10 are trademarks from Mitsui.

The method of the invention applies to any polythiourethane, regardless of the specific application for which it was conceived. Advantageously, the polythiourethane to be degraded according to the method of the invention can be derived from optical lenses.

The polythiourethane may contain at least one additive agent such as an internal release agent, a reaction catalyst, a heat stabilizer, a UV absorber, a blue cut agent, optionally associated with a color balancing agent, a bluing agent, a mold lubricant, a colorant, an anti-oxidant, a coloration inhibitor, a fluorescent brightener and the like, depending on the objective.

The polymer according to the invention identifies an article comprising or deriving from domestic, commercial or industrial mixed or sorted waste streams or collected plastic wastes and it can be characterized by any physical conformation, e.g. sheets, films, tubes, pipes, straws, rods, ropes, strings, lines, nets, reticulated sheets, three dimensional regular shapes, blocks, sheaths, fibers, membranes, woven and non-woven textiles, bags, containers, bottles, capsules, packaging, microspheres, (electro)mechanical components, clothing, coatings and panels.

As used herein, the terms "enzymatic degradation", "polymer degradation", "depolymerization" and "enzymatic hydrolysis" are used interchangeably and they refer to a chemical process wherein a polymeric material undergoes degradation by one or more degradative enzymes, which cleave one or more chemical bonds between the repeating units of the polymer. The result of the enzymatic degradation is degradation products, which are the repeating units, monomers,

oligomers and/or fragments of the polymer.

The terms "complete degradation" and "complete depolymerization" mean that the polymer according to the invention is entirely converted into the repeating unit(s) of which it is composed. On the contrary, the terms "partial degradation" and "partial depolymerization" mean that the polymer according to the invention is only partly converted into the repeating unit(s) of which it is composed, i.e. a part of the polymer is converted into oligomers and/or fragments.

As used herein, the term "degradative enzyme" refers to an enzyme capable of degrading a polythiourethane. The degradative enzyme can hydrolyze the thiourethane bond, or the ester bond, or both of them.

According to a first embodiment, the invention concerns a method for the enzymatic degradation of a polythiourethane, comprising contacting said molecule with at least one enzyme capable of degrading said PTUR and selected from the group consisting of:

- enzyme 1, the amino acid sequence of which presents at least 60% identity with the following amino acid sequence:

MRVSLRSITSLLAAATAAVLAAPATETLDRRAALPNPYDDPFYTTPSNIGTFAKGQVIQS
RKVPTDIGNANNAASFQLQYRTTNTQNEAVADVATVWIPAKPASPPKIFSYQVYEDA
TALDCAPSYSYLTGLDQPNKVTAVLDTPIIIGWALQQGYYVVSSDHEGFKAAFIAGYE
EGMAILDGIRALKNYQNLPSDSKVALEGYSGGAHATVWATSLADSYAPELNIVGASH
GGTPVSAKDTFTFLNGGPFAGFALAGVSGLSLAHPDMESFIEARLNAKGQQTLKQI

RGRGFCLPQVVLTYPFLNVFSLVNDTNLLNEAPIAGILKQETVVQAEASYTVSVPKFP
RFIWHAIPDEIVPYQPAATYVKEQCAKGANINFSPYPIAEHLTAEIFGLVPSLWFIKQA
FDGTTPKVICGTPIPAIAGITTPSADQVLGSDLANQLRSLNGKQSAFGKPFGPITPP
(SEQ ID NO: 1 in the annexed sequence listing),

- enzyme 2, the amino acid sequence of which presents at least 60% identity with the following amino acid sequence:

SEVCFPRLGCFSDDAPWAGIVQRPLKILPWSPKDVDTRFLLYTNQNQNNYQ
ELVADPSTITNSNFRMDRKTRFIIHGFIDKGEEDWLSNICKNLFKVESVNCICVDWKG
GSRTGYTQASQNIRIVGAEVAYFVEVLKSSLGYSPSNVHVIGHSLGSHAAGEAGRR
TNGTIERITGLDPAEPCFQGTPELVRLDPSDAKFVDVIHTDAAPIIPNLGFGMSQTVG
HLDFFPNGGKQMPGCQKNILSQIVDIDGIWEGTRDFVACNHLRSYKYYADSILNPDG
FAGFPCDSYNVFTANKCFPCPSEGCPQMGHYADRFPGKTNGVSQVFYLNTGDAS
NFARWRYKVSVTLSGKKVTGHILVSLFGNEGNSRQYEIYKGTLQPDNTHSDEFDSD
VEVGDLQKVKFIWYNNNVINPTLPRVGASKITVERNDGKVYDFCSQETVREEVLLTL
NPC (SEQ ID NO: 2 in the annexed sequence listing),

- enzyme 3, the amino acid sequence of which presents at least 60% identity with the following amino acid sequence:

MWLLPLVLTSLASSATWAGQPASPPVVDTAQGRVLGKYVSLEGLAQPVAVFLGVPFAKPPLGSLRFAPPQPAEPWSFVKNTTSYPPMCCQDPVVEQMTSDLFTNGKERLTLEFSEDCLYLNIYTPADLTKRGRLPVMVWIHGGGLVLGGAPMYDGVVLAAHENVVVVAIQYRLGIWGFFSTGDEHSRGNWGHLDQVAALHWVQENIANFGGDPGSVTIFGESAGGESVSVLVLSPLAKNLFHAISESGVALTVALVRKDMKAAAKQIAVLAGCKTTTSAVFVHCLRQKSEDELLDLTLKMKFLTLDFHGDQRESHPFLPTVVDGVLLPKMPEEILAEKDFNTVPYIVGINKQEFGWLLPTMMGFPLSEGKLDQKTATSLLWKSYPIANIPEELTPVATDKYLGGTDDPVKKKDLFLDLMGDVVFGVPSVTVARQHRDAGAPTYMYEFQYRPSFSSDKKPKTVIGDHGDEIFSVFGFPLLKGDAPEEEVSLSKTVMKFWANFARSGNPNGEGLPHWPMYDQEEGYLQIGVNTQAAKRLKGEEVAFWNDLLSKEAAKKPPKIKHAEL (SEQ ID NO: 3 in the annexed sequence listing),

- enzyme 4, corresponding to a lipase from *Aspergillus oryzae,*
- enzyme 5 corresponding to a lipase from *Thermomyces lanuginosus,*
- enzyme 6 corresponding to a serine endopeptidase,
- enzyme 7, corresponding to a protease from *Bacillus licheniformis,*
- enzyme 8, the amino acid sequence of which presents at least 60% identity with the following amino acid sequence:

MTELTVQTRCGALKGTAGHGVRTWKSIPYAKPPVGELRFKAPEPPVPWDGVKNADSFGPVCPQPADLLSMSFSGDVPPQSEDCLYLNVFAPDSEGGKRPVMVWIHGGAFFLGAGSEPTYDASALAADGDVIVVTLNYRLGPFGFLHLFSIDDTYPGNIGMLDQIAALRWVKDNISAFGGDPDNVTVFGESAGGMSIASLMAMPDAKGLFHKAILESGASQTMTADVAKEITTAFIQEAGTDQLQELSVNDILKTADKLRNTIDQSIFHLLFQPAIDPATLPAEPAKAIADGAAEGIPMIIGTNRDEAYLFFTPDTDIHSEKKQQEYLLYHLGENSAKLAADLYPHSLTGQIDMMTDLKFWRPAVAFAQEQSQYAPVWMYRFDWHGETPPFHKAVHALELPFVFGNFDSLKKTLKEPLSEDVKQLSKLIQSAWIAFAKTGKPDTDQLHWPQYETGSRETIIFNTSVSTESDPDSAKRRILFQA (SEQ ID NO: 4 in the annexed sequence listing),

- enzyme 9, the amino acid sequence of which presents at least 60% identity with the following amino acid sequence:

MSASSLKFPIVLVHGLLGFDKIGGIYPYFYGIKEALEKAGAKVYIATLSALNSNELRGEQLLEFVRKVQAETGAAKVNLIGHSQGPLACRYVAATHPELIASVTSVNGVNHGSEVADLVRLALTPGRLPESIANAAMSAFGQLLSALAGSPRLPQSGIEALEALTSEGVAAFNKKYPQGLPAEWGGEGKELVNGVYYYSWSGIIDYNPLHQGANNLDPLHVAMLAFSILFTNERFQNDGLVGRYSSHLGKVIGSDYSMDHVDAINQLAGVVANNTDPVQLFVEHVARLKSKGL (SEQ ID NO: 5 in the annexed sequence listing),

- enzyme 10, the amino acid sequence of which presents at least 60% identity with the following amino acid sequence:

MEHGNRRGPGRACAHQDTVTLKAGTRRSTSAIARTAAGTAAAIAAAIAMAW PASATASSDYAKTRYPIVLVHGLTGAAKMGGVIDYWYGIPEVLRANGATVYVSTVPS FNSDEERALALQAYVRAVKLETGADKVNLIGHSQGGPTSRMLAAMSPQDVASVTTI GSPHRGSQLADVVLDVINGISSIPLAGPALVNVIQGVTNLLGWFNGLFNGQALNQDA IASARTLTSQGATEQNARLNATLAPGSQSALGPDCDTPGAVAEQRQGRDASGKLV TYTQAAYSWTGRGGPINLLRSNLLDPSTAAMAAAAGVMGMKGAGPNDGMVSVCS AKWGRVLSTDYYWNHLDEINQMLGMYQDVDPRTVILNHANRLRNDQL (SEQ ID NO: 6 in the annexed sequence listing),

- enzyme 11, the amino acid sequence of which presents at least 60% identity with the following amino acid sequence:

MKKLVALLIVGLFVSISLTGLGLAGGTAPYKCDTRYPVVLAHGMGASTKILGIV DYWYGIEDALKAEGASVYFTSVNAMGSTVDKAADFKQQFMEILAVTGAPKANIIGHS HGTLYTRYAISNLGLAPYVASYTSLAGPHRGSAVASLIMYDLPDWLLAAGGDVLNFV YTFIFGDTNPDSLQNALDLCPDYMVNTFNPNTPNIPGIYYQSWAAKAKTSCPSVILEP TWLIMLIEEGANDGLVSVESAKWGNFRGVEDAAWYSAGCDHLNIVGQLFGVTPGF DAPQFFVDIVEDLKGRGY (SEQ ID NO : 7 in the annexed sequence listing).

On one hand, hydrolytic enzymes or hydrolases capable to cleave/hydrolyse the carbonate function such as lipases, proteases, polyurethanases, cutinases, and esterases and, on the other hand, laccases are designated as degradative enzymes in the sense of the present invention.

The degradative enzymes selected from the group consisting of enzymes 1, 2, 3, 6, 8, 9, 10 and 11 may be of any suitable origin, such as vegetable, animal, bacterial, fungal and yeast origin. Origin can further be mesophilic or extremophilic (psychrophilic, psychrotrophic, thermophilic, barophilic, alkalophilic, acidophilic, halophilic, *etc.*). In general, any microorganism capable of expressing the enzymes disclosed herein, whether naturally or by recombinant DNA techniques and recombinant protein expression methods, may be suitable to carry out the method of the invention. Preferably, the degradative enzymes originate from mesophilic organism.

The enzymes implemented in the invention can be conveniently produced by recombinant techniques using a cellular expression system. Any suitable expression system may be used, including those involving prokaryotic cells and eukaryotic cells. Suitable prokaryotic cells may be any Gram-positive or Gram-negative bacterium. Gram-positive bacteria include, but are not limited to, *Bacillus, Clostridium, Corynebacterium, Enterococcus, Geobacillus, Lactobacillus, Lactococcus, Oceanobacillus, Staphylococcus, Streptococcus,* and *Streptomyces.* Gram-negative bacteria include, but are not limited to, *Acinetobacter, Campylobacter, E. coli, Flavobacterium, Fusobacterium, Helicobacter, Ilyobacter, Neisseria, Pseudomonas, Salmonella,* and *Ureaplasma.* Particularly preferred as a heterologous host are E. *coli* strains engineered to export the enzymes to the cell surface or extracellularly. Suitable eukaryotic cells may be selected from: yeasts, fungi, insect cells, mammalian cells, and plant cells. Suitable yeast cells may be selected from: *Candida, Hansenula, Kluyveromyces, Pichia, Saccharomyces* such as *Saccharomyces cerevisiae, Schizosaccharomyces* such as *Schizosaccharomyces pombe,* or *Yarrowia* cell. Suitable fungal cells may be selected from a filamentous fungus, for example *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* and *Trichoderma.*

As used herein, the term "recombinant" refers to any type of material that has been produced by any of the techniques of recombinant DNA technology, for example using recombinant nucleic acids, Polymerase Chain Reaction (PCR), *etc.* Recombinant proteins are proteins produced by the expression of recombinant nucleic acids and the enzymes disclosed in the present Application can be recombinant enzymes.

The enzymes implemented in the present invention can equally be produced using other techniques known in the art, including direct modifications of isolated enzymes and direct protein synthesis.

Alternatively, the enzymes implemented in the present invention can also be commercially available and used according to the manufacturer's instructions.

As used herein, the terms "sequence identity" or "identity" in relation to an amino acid sequence define the

number (or fraction, when expressed as a percentage, %) of identical amino acid residues between two polypeptide sequences. The sequence identity is determined by comparing the sequences when aligned so as to maximize overlap and identity while minimizing sequence gaps, and may be determined using any of a number of alignment algorithms known to those skilled in the art. In particular, sequence identity may be determined using computer programs (such as the BLASTP program publicly available from NCBI and other sources) or direct sequence comparison.

According to a specific aspect, the amino acid sequences of the enzymes implemented in the invention is at least about 65%, 70%, 75%, 80%, 85%, 90%, 95%, 96%, 97%, 98%, 99% or even 100% identical to the amino acid sequences set forth in the sequences provided herein and possess the same enzymatic function.

In other terms, such enzyme variants retain the enzymatic activity of the enzymes whose sequence is set forth in the sequences provided herein, and include mutants differing by the addition, deletion or substitution of one or more amino acid residues. The variant may have one or more conservative changes, wherein a substituted amino acid has similar structural or chemical properties (e.g., replacement of leucine with isoleucine). Alterations may occur at the amino- or carboxy-terminal positions of the reference polypeptide sequence or anywhere between those terminal positions, interspersed either individually among the amino acids in the reference sequence or in one or more contiguous groups within the reference sequence.

In a particular embodiment, the amino acid sequence of enzyme 1 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 1.

Advantageously, the amino acid sequence of enzyme 1 used in the method according to the invention comprises or consists of SEQ ID NO: 1.

In a more particular embodiment, the enzyme 1 as implemented in the invention is a lipase A and, preferably, a lipase A from *Candida antarctica.*

In an even more particular embodiment, the enzyme 1 as implemented in the invention is the commercial lipase Novocor® AD-L. In other words, the enzyme 1 implemented in the present invention is commercially available under the name Novocor® AD-L, which is produced and supplied by, for example, Strem Chemicals, Inc. or Sigma Aldrich (Merck).

As used herein, the term "commercial" and the expression "commercially available" refers to an enzyme that is produced by a company and that can be purchased from said company in an essentially pure form and with a given activity, measured in U, where U is the unit of enzyme's catalytic activity, and 1U is defined as the amount of the enzyme that catalyzes the conversion of one micromole of substrate per minute under the specified conditions of the assay method.

The enzyme 1 as implemented in the invention is capable of hydrolyzing the thiourethane bond as demonstrated by the formation, among the degradation products, of m-xylylenediamine (m-XDA), which is a signature of the hydrolysis of thiourethane bond.

Consequently, the enzyme 1 as implemented in the invention is capable of hydrolyzing the thiourethane bond in a polythiourethane and can be used to this aim to hydrolyze the thiourethane bond present in a molecule, where such molecule can be any molecule (monomer, polymer, *etc...*) containing at least one thiourethane bond.

In a particular embodiment, the amino acid sequence of enzyme 2 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 2.

Advantageously, the amino acid sequence of enzyme 2 used in the method according to the invention comprises or consists of SEQ ID NO: 2.

In a more particular embodiment, the enzyme 2 as implemented in the invention is a pancreatic lipase, and preferably a porcine pancreatic lipase.

In an even more particular embodiment, the enzyme 2 as implemented in the invention is a commercial porcine pancreatic lipase. For example, the enzyme 2 implemented in the present invention is the porcine pancreatic lipase commercially available under the product number "L3126", which is produced and supplied by Sigma-Aldrich (Merck).

The enzyme 2 as implemented in the invention is capable of hydrolyzing the thiourethane bond as demonstrated by the formation, among the degradation products, of m-xylylenediamine (m-XDA), which is a signature of the hydrolysis of thiourethane bond.

Consequently, the enzyme 2 as implemented in the invention is capable of hydrolyzing the thiourethane bond in a polythiourethane and can be used to this aim to hydrolyze the thiourethane bond present in a molecule, where such molecule can be any molecule (monomer, polymer, *etc...*) containing at least one thiourethane bond.

In a particular embodiment, the amino acid sequence of enzyme 3 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 3.

Advantageously, the amino acid sequence of enzyme 3 used in the method according to the invention comprises or consists of SEQ ID NO: 3.

In a more particular embodiment, the enzyme 3 as implemented in the invention is an esterase, preferably an esterase from porcine liver and more preferably an esterase from porcine liver the CAS Number of which is 9016-18-6.

In an even more particular embodiment, the enzyme 3 as implemented in the invention is a commercial esterase from porcine liver. For example, the enzyme 3 implemented in the present invention is the esterase from porcine liver commercially available under the product number "E3019", which is produced and supplied by Sigma-Aldrich (Merck).

In a particular embodiment, the enzyme 4 as implemented in the invention is a *Aspergillus oryzae* lipase the CAS Number of which is 9001-62-1. In a more particular embodiment, the enzyme 4 as implemented in the invention is a commercial *Aspergillus oryzae* lipase.

In an even more particular embodiment, the enzyme 4 as implemented in the invention is the commercial *Aspergillus oryzae* lipase commercially available under the product number "L0777", which is produced and supplied by Sigma Aldrich (Merck).

In another even more particular embodiment, the enzyme 4 as implemented in the invention is the commercial lipase Novozym® 51032 from *Aspergillus oryzae.* In other words, the enzyme 4 implemented in the present invention is commercially available under the name Novozym® 51032, which is produced and supplied by, for example, Novozymes A/S or Strem Chemicals, Inc.

In a particular embodiment, the enzyme 5 as implemented in the invention is a lipase from *Thermomyces lanuginosus.*

In a more particular embodiment, the enzyme 5 as implemented in the invention is the commercial lipase Lipozyme® TL 100L. In other words, the enzyme 5 implemented in the present invention is commercially available under the name Lipozyme® TL 100L, which is produced and supplied by, for example, Strem Chemicals, Inc or Sigma Aldrich (Merck).

In a particular embodiment, the enzyme 6 as implemented in the invention is a serine endopeptidase and preferably a commercial serine endopeptidase.

In a more particular embodiment, the enzyme 6 as implemented in the invention is the commercial Savinase® 16L. In other words, the enzyme 6 implemented in the present invention is commercially available under the name Savinase® 16L, which is produced and supplied by, for example, Novozymes A/S or Strem Chemicals, Inc.

In a particular embodiment, the enzyme 7 as implemented in the invention is a protease from *Bacillus licheniformis*.

In a more particular embodiment, the enzyme 7 as implemented in the invention is a commercial protease from *Bacillus licheniformis.* For example, the enzyme 7 implemented in the present invention is the protease from *Bacillus licheniformis* commercially available under the product number "P4860", which is produced and supplied by Sigma-Aldrich (Merck).

In a particular embodiment, the amino acid sequence of enzyme 8 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 4.

Advantageously, the amino acid sequence of enzyme 8 used in the method according to the invention comprises or consists of SEQ ID NO: 4.

According to an even more particular embodiment, enzyme 8 belongs to the enzyme family of carboxylesterases/lipases.

In a particular embodiment, the amino acid sequence of enzyme 9 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 5.

Advantageously, the amino acid sequence of enzyme 9 used in the method according to the invention comprises or consists of SEQ ID NO: 5.

According to an even more particular embodiment, enzyme 9 belongs to the enzyme family of putative lactonizing lipases.

In a preferred embodiment, enzyme 9 as defined according to any of the embodiments above, is used in the method of the invention under the form of purified protein or crude lysate.

In a particular embodiment, the amino acid sequence of enzyme 10 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 6.

Advantageously, the amino acid sequence of enzyme 10 used in the method according to the invention comprises or consists of SEQ ID NO: 6.

According to an even more particular embodiment, enzyme 10 belongs to the enzyme family of triglyceride lipases.

In a particular embodiment, the amino acid sequence of enzyme 11 as implemented in the invention exhibits at least 60% identity and can exhibit at least 65%, 70%, 75%, 80%, 85%, 90%, 95%, 96%, 97%, 98%, 99% or even 100% identity with the amino acid sequence SEQ ID NO: 7.

Advantageously, the amino acid sequence of enzyme 11 used in the method according to the invention comprises or consists of SEQ ID NO: 7.

According to an even more particular embodiment, enzyme 11 belongs to the alpha/beta hydrolase superfamily.

In a preferred embodiment, enzyme 11 as defined according to any of the embodiments above, is used in the method of the invention under the form of a crude lysate.

According to the invention, two or more enzymes as above defined (*i.e.* enzymes 1-11) can be used at the same time for enzymatic degradation of the PTUR. The two or more enzymes can interact in the reaction mixture and the result of their interaction can be additive (*i.e.*, the degradative activity of the enzymes is the sum of the activities of the enzymes if they were acting separately) or synergistic (*i.e.*, the degradative activity of the enzymes is more than the sum of the activities of the enzymes if they were acting separately). When three or more enzymes as above defined (*i.e.* enzymes 1-11) are present, both additive and synergistic effects can be at play at the same time. Other enzyme(s) can also be present in the reaction mixture, in addition to the enzyme(s) disclosed in the present invention.

In the invention, the polythiourethane to be degraded can be implemented in the form of a mixture of two or more polythiourethanes.

Before enzymatic degradation according to the method of the invention, the polythiourethane can be submitted to a pre-processing step aiming at promoting or accelerating the enzymatic degradation by altering the physical state of the polymer. This pre-processing step physically changes the structure of the polymer, for example by increasing the surface area available for contact with the degradative enzyme.

Pre-processing is preferably conducted before, or during, contact with the enzyme(s) as implemented in the invention, *i.e.* as a pre-treatment or as a co-processing step. Preferably, pre-processing is conducted as a pre-treatment processing step.

Pre-processing may, for example, comprise mechanical processing of the polymer by a treatment selected from: washing, centrifugation, cleaning, collision, sorting, grinding, homogenization, shredding, cutting, impacting, crushing, shearing, shredding, rotary drum tumbling, fractionation, sonication, melting, extrusion, spinning, liquefaction, maceration, micronization, pelletization, screw pressing, piston pressing and granulation, or a combination of two or more of the preceding methods. Grinding is particularly preferred, optionally together with one or more of the other pre-processing steps listed above.

Alternatively, or in addition, the PTUR may be physically processed by a treatment selected from: irradiation, for example drying, UV irradiation, amorphization, agglomeration, heating (e.g. by microwaves), cooling, freezing, dessication, or a combination of two or more of the preceding methods.

In the method according to the Invention, the PTUR can be put in contact with at least one enzyme as above defined in any appropriate way, *i.e.* in any way that allows the enzyme(s) to interact with the PTUR so that it can exert its degrading activity. Non-limiting examples of contacting the enzyme(s) and polymer are the followings:

- the PTUR can be dispersed in the solution containing the enzyme(s). In this case, the polymer can be in any form, e.g., it can be added as a film, sheet or as a granular material. Other polymer forms, e.g. mouldings, can be added as an integral whole or in comminuted form. Coated or adhesively bonded materials, or materials on which coatings of the polymer have been deposited, such as paper or cardboard as well as coated paper or coated cardboard, can be added as an integral whole or in comminuted form to the enzyme-containing solution;

- the enzyme solution can be sprayed onto the PTUR: the aqueous enzyme-containing solution can be sprayed or deposited by spraying onto the polymer to be degraded. In this case, the enzyme solution should completely cover the polymer, whatever its form;

- the PTUR may be contacted directly with the enzyme-expressing organism. Unpurified (cell lysates) or semi-purified culture supernatants containing the enzyme(s) may also be contacted with the polymer.

Hence, the PTUR may be present in bulk or as a solution, preferably as an aqueous solution or water. The aqueous solution which is required for carrying out the method according to the invention can be buffered. The pH and temperature depend on the specific enzyme(s) used, as the activity of each enzyme is optimal at different pH and temperature values. The pH is generally between 2 and 12, preferably between 5 and 9 and most preferably between 6 and 8. The temperature at which enzymatic degradation is carried out is generally between 5°C and 60°C; it is preferably between 20°C and 50°C, and most preferably between 30°C and 50°C. Non aqueous solvents/water mixtures can also be used as solvents. Non aqueous solvents can be organic solvents, deep eutectic solvents or natural deep eutectic solvents.

The following are examples of buffers which can be used according to the invention: citrate, acetate, phosphate, formate, carbonate, tris-hydroxymethylaminomethate, triethanolamine, imidazole, oxalate, tartrate, fumarate, maleate, phthalate, succinate and ethylenediamine, as well as a plurality thereof. Acetates and phosphates are preferably used as buffers.

The PTUR may be contacted with the enzyme(s) as above-defined at a concentration and temperature for a time sufficient to achieve the desired amount of degradation. Suitable times range from a few hours to several days and may be

selected to achieve a desired amount of conversion. Examples of reaction times include 1 day, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 days. In some embodiments, reaction times may be one or more weeks. Those skilled in the art will be able to determine an appropriate reaction time by reference to the physical state of the polymer, the enzyme(s) selected and the extent of degradation required.

The method can comprise agitating the reactants to improve contact between the enzyme(s) and the polymer and so promote adsorption of the enzyme(s) onto the PTUR. This step may comprise continuous stirring, for example at a rate of between 100 revolutions per minute (rpm) and 5000 rpm.

Chemical elements and/or compounds that may be necessary for the enzymatic reaction or that may have a positive influence on it, can also be added to the reaction mixture. Preferably, the chemical elements and/or compounds are selected and added to the reaction mixture for their positive impact on enzyme activity. Examples of chemical elements and/or compounds with positive impact on enzyme activity include, but are not limited to, metal ions (e.g., sodium or calcium ions) and salts (added, e.g., to regulate the ionic strength of the solution).

The whole process can be carried out in any appropriate container, for example a large reactor vessel comprising a mixer and surrounded by a heating mantle to regulate the temperature.

The concentration of the enzyme should suffice to quantitatively degrade the polymer. This degradation may be partial or complete. The amount of enzyme(s) required to carry out the method of the invention can be readily determined by the skilled person by reference, *inter alia,* to the relative amount of the polymer and/or the number of any additional enzyme present in the reaction mixture.

For example, in preferred embodiments, the enzyme(s) as implemented in the invention is used in an amount up to 5% by weight of the PTUR, more preferably up to 1%, even more preferably up to 0.1%, and yet more preferably up to 0.05% by weight of the polymer. For example, the amount of enzyme of the invention may be in a range of 0.001 % to 5% by weight of the polymer, preferably in the range of 0.002% to 1%, more preferably in the range of 0.003% to 0.1%, and even more preferably in the range of 0.005% to 0.05% by weight of the polymer.

According to another embodiment of the method according to the present invention, the at least one degradative enzyme as previously defined is used in the form of a pure protein, a crude lysate or immobilized on a support.

Herein, "in pure form" is used to mean in "substantially pure form", i.e., a purity that allows for the effective use of the protein in the method described herein. For a protein to be most useful in the method of the invention or in any method utilizing enzymes described herein, it is most often substantially free of contaminants, other proteins and/or chemicals that might interfere or that would interfere with its use in the method (e.g., that might interfere with enzyme activity). This form can also be known as "cell-free lysate" which is obtained after centrifuging and filtering the lysate obtained during classical protein over-expression.

As used herein, the expression "substantially pure form" means that the enzyme should be at least 90% pure, preferably 95% pure and even more preferably 98% pure.

As used herein, the expressions "crude lysate" or "cell lysate" are equivalent and can be used interchangeably. Both expressions refer to a specific product obtained during classical protein over-expression.

In particular, a crude lysate is what is obtained when the host cells (e.g., *Escherichia coli* cells), after they have been allowed to over-express the protein of interest in culture media, are lysed with any of the methods known in the art, with the aim to liberate in the supernatant the soluble over-expressed protein that is present in the cell.

Different methods can be used depending on the type of host cells and on the quantity of host cells to be lysed. Examples of methods for cell lysis comprise liquid shear pressure techniques (e.g., French press and homogenizer), ultrasonication, freeze and thawing (coupled with enzymatic lysis, e.g. with lysozyme), glass beads, osmotic shock and chemical lysis (e.g., by using detergents).

Despite containing the protein in a non-pure form, because all cell debris and chemicals are still present, in certain cases cell lysates can effectively be used in place of the enzymes in their pure form. This can be the case when, for example, the concentration of the enzyme is particularly high. The main advantage in the use of cell lysates is that this avoids further steps of protein purification, which requires additional time and which increases the costs of the degradation process.

According to the invention, the enzyme(s) may be present in the composition in an immobilized form. Immobilization is often implemented to improve the stability of the enzyme(s). In this case, the enzyme has been immobilized on a suitable support by means of any immobilization method. For example, the enzyme can be immobilized onto solid supports, matrices or particles (for example selected from glass beads and mineral, polymer or metallic particles or matrices).

The immobilization method is based either on physical techniques such as adsorption, entrapment, and encapsulation or on chemical bonding processes such as covalent bonding and crosslinking.

The reaction products of the enzymatic degradation carried out according to the method of the invention comprise one or more compounds selected from a compound presenting at least a thiol function, a compound presenting at least an amine function, a compound presenting at least one carboxylic function, a compound presenting at least an

alcohol function and $CO_2$. One or more of these compounds can be identified by suitable analytical techniques to evaluate the course of the reaction. Hence, the identification of one or more of such compounds advantageously allows establishing whether the enzymatic degradation has taken place or is taking place.

The compound presenting at least a thiol function can conveniently be measured by, for example, High-performance liquid chromatography (HPLC) after or not derivatization with benzoyl chloride (BzCl) or using the Ellman's assay, based on the use of the Ellman's reagent (5,5'-dithiobis-(2-nitrobenzoic acid or DTNB). Other techniques known to the skilled person may equally be used.

The compound presenting at least an amine function can conveniently be detected by, for example, High-performance liquid chromatography (HPLC) after or not derivatization with benzoyl chloride (BzCl). Other techniques known to the skilled person may equally be used.

The compound presenting at least one carboxylic function can conveniently be detected by, for example, Bromothymol blue assay (described in the experimental part below). Other techniques known to the skilled person may equally be used.

The compound presenting at least an alcohol function can conveniently be detected by, for example, Gas Chromatography-Mass Spectrometry (GC-MS) or HPLC-MS. Other techniques known to the skilled person may equally be used.

$CO_2$ can conveniently be detected by, for example, Bromothymol blue assay. Other techniques known to the skilled person may equally be used.

It will be appreciated that the enzymatic degradation by the enzymes according to the method of the invention need not completely depolymerize the polymer, and that the nature and extent of the degradation may be determined, *inter alia,* by the enzyme selected, the reaction conditions and the chemical nature and/or physical form of the polymer.

More particularly, factors that control the rate at which the polymeric material degrades include the concentration of the enzyme, the enzyme kinetics with respect to polymer degradation, the polymer matrix, the polymer shape (e.g., changing the shape may affect the rate of substrate transport into and out of the material), the environment in which the reaction takes place (e.g., the environment can vary in temperature, pH, ionic strength and other factors that alter enzyme activity) and the fluid dynamics of the outer environment. Other factors that can also alter the rate of polymer degradation are readily available to those with ordinary skill in the art.

From the above, it is thus apparent that the enzymatic degradation mediated by enzymes according to the invention may yield fragments, monomers and/or oligomers of the PTUR.

Consequently, the method according to the present invention may further comprise the step of fractionating, isolating or purifying degradation product(s) obtained. Exemplary methods for purification/isolation/separation of degradation products comprise: filtration, distillation, solvent extraction (e.g., liquid/liquid extraction), precipitation, crystallization, evaporative concentration, evaporative crystallization, affinity chromatography, ion exchange chromatography, solvent extraction, centrifugation, electrophoresis, electrodialysis, hydrophobic interaction chromatography, gel filtration chromatography, reverse phase chromatography, differential solubilization, high performance liquid chromatography (HPLC), and/or reversed-phase HPLC and combinations of two or more of the preceding methods.

The reaction product(s) may also be recovered and re-cycled for obtaining other compounds of interest.

Thus the present invention concerns the use of the method as previously defined for producing fragments, oligomers, monomers or repeating units from a polythiourethane that can be recovered and re-cycled for obtaining the original polythiourethane.

According to another embodiment, the invention also concerns a kit for the enzymatic degradation of a polythiourethane, comprising at least one enzyme as previously defined.

According to another embodiment, the kit further comprises at least one reagent for the degradation reaction.

The kit according to the present Invention can optionally include at least one other reagent required to conduct the enzymatic degradation such as buffers, salts, enzyme co-factors and the like. The kit may additionally include one or more controls.

Other components may also be included in the kit. The various components of the kit are optionally provided in suitable containers. The kit can further include containers for holding or storing the polymer. Where appropriate, the kit may also optionally contain reaction vessels, mixing vessels and other components that facilitate the preparation of reagents and/or the polymer.

Other features and advantages of the present invention will become apparent from the following detailed description, which makes reference to the accompanying figures.

## FIGURE LEGENDS

**Figure 1: Determination of the degrading activity of some of the claimed commercial enzymes of the invention**

**towards Substrate A.** Determination of the degrading activity of enzymes 1, 2, 3, 4(1), 4(2), 5, 6 and 7 towards Substrate A by means of a Bromothymol blue (BTB) high-throughput assay. Substrate control (SC) means that the optical density (OD) of the substrate only has been measured (*i.e.*, in the absence of any enzyme). All reactions and spectrophotometric measurements have been carried out in the same experimental conditions, *i.e.,* solvent, buffer, BTB, temperature, *etc,* as detailed in the Materials & Methods section. In each case, the OD has been measured immediately after mixing all the reagents in the appropriate conditions (t = 0h) as well as after 24h (t = 24h).

**Figure 2: Determination of the degrading activity of some of the claimed commercial enzymes of the invention towards Substrate B.** Determination of the degrading activity of enzymes 1, 2, 3, 4(1), 4(2), 5, 6 and 7 towards Substrate B by means of a Bromothymol blue (BTB) high-throughput assay. Substrate control (SC) means that the optical density (OD) of the substrate only has been measured (i.e., in the absence of any enzyme). All reactions and spectrophotometric measurements have been carried out in the same experimental conditions, *i.e.,* solvent, buffer, BTB, temperature, *etc,* as detailed in the Materials & Methods section. In each case, the OD has been measured immediately after mixing all the reagents in the appropriate conditions (t = 0h) as well as after 24h (t = 24h).

**Figure 3: Determination of the degrading activity of the claimed 'in-house' enzymes of the invention towards Substrate A.** Determination of the degrading activity of enzymes 8, 9, 10 and 11 towards Substrate A by means of a Bromothymol blue (BTB) high-throughput assay. Substrate control (SC) means that the optical density (OD) of the substrate only has been measured (*i.e.*, in the absence of any enzyme). As a positive control (PC), the degrading activity of enzyme 4(2) towards the substrate has been measured. All reactions and spectrophotometric measurements have been carried out in the same experimental conditions, i.e., solvent, buffer, BTB, temperature, *etc,* as detailed in the Materials & Methods section. In each case, the OD has been measured immediately after mixing all the reagents in the appropriate conditions (t = 0h) as well as after 24h (t = 24h).

**Figure 4: Determination of the degrading activity of the claimed 'in-house' enzymes of the invention towards Substrate B.** Determination of the degrading activity of enzymes 8, 9, 10 and 11 towards Substrate B by means of a Bromothymol blue (BTB) high-throughput assay. Substrate control (SC) means that the optical density (OD) of the substrate only has been measured (*i.e.,* in the absence of any enzyme). As a positive control (PC), the degrading activity of enzyme 4(2) towards the substrate has been measured. All reactions and spectrophotometric measurements have been carried out in the same experimental conditions, *i.e.,* solvent, buffer, BTB, temperature, *etc,* as detailed in the Materials & Methods section. In each case, the OD has been measured immediately after mixing all the reagents in the appropriate conditions (t = 0h) as well as after 24h (t = 24h).

## DETAILED DESCRIPTION OF PARTICULAR EMBODIMENTS

### Materials & Methods

***Bromothymol blue (BTB) high-throughput assay for screening and selection of the enzymes with the highest degrading activity using two model substrates referenced* as *"Substrate A" and "Substrate B"***

In order to determine the enzymes with the highest degrading activity, a high-throughput BTB assay was used. This assay makes use of bromothymol blue, which is a pH indicator and therefore changes its color as a function of the pH of the reaction mixture. In the presence of a degradative enzyme, the substrate is degraded in various products, including $CO_2$ and 3-mercaptopropanoic acid (which are released after hydrolysis of the ester bond in Substrate A and Substrate B), which have the effect to decrease the pH of the reaction mixture. For $CO_2$, this is explained by the formation of carbonic acid as indicated in the equation below. This assay thus allows to easily detecting the $CO_2$ and 3-mercaptopropanoic acid released during enzymatic degradation.

$$CO_{2(aq.)} \rightleftharpoons \underset{\text{Carbonic acid}}{H_2CO_3} \rightleftharpoons \underset{\text{Bicarbonate ions}}{HCO_3^-} + H^+$$

As the enzymatic degradation of PTURs is a slow process, model substrates similar in structure and functional groups to PTURs were used to screen the numerous enzymes in a short time period. The chosen model substrates are Substrate A and Substrate B. The chemical formula of Substrate A is:

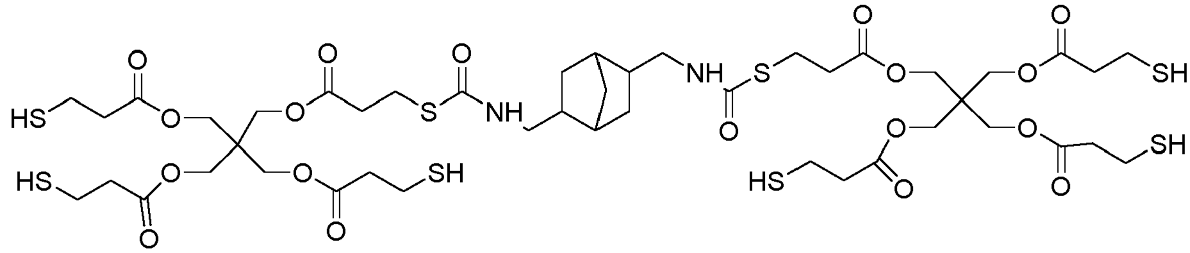

And the chemical formula of Substrate B is:

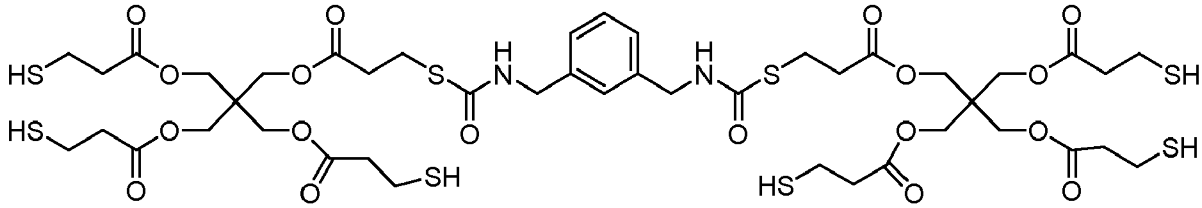

Various parameters of the BTB assay were optimized such as absorption maxima, BTB concentration range, concentration of the buffer to be used *etc.* (Shimada and Hasegawa, 2017). After optimization of these parameters, a bicarbonate calibration curve was obtained (Ocal *et al.,* 2021). This calibration curve obtained with optimized parameters (5 mM, sodium phosphate buffer, pH = 7.0, BTB concentration of 135 μM) was used to evaluate the degrading activity of the enzymes towards Substrate A and Substrate B.

In addition to the BTB assay, high throughput screening of the enzymes may be carried out by using Ellman's reagent based high throughput assay for free thiol estimation as well as HPLC-UV based detection after BzCl derivatization for the estimation of primary amines.

The Table 1 lists the 11 enzymes with the highest degrading activity towards Substrate A and Substrate B identified using the BTB assay.

**Table 1: Enzymes with highest degrading activity towards Substrate A and Substrate B as determined by BTB high-throughput assay. 'In-house' means that the enzyme has not been purchased but has been cloned, expressed and purified by the inventors.**

| Enzyme as previously defined | Origin | Details |
|---|---|---|
| Enzyme 1 | Commercial | Lipase A from *Candida Antarctica* (CAL-A) (commercial lipase Novocor® AD-L) |
| Enzyme 2 | Commercial | Commercial Porcine Pancreatic Lipase (PPL) (L3126, Sigma-Aldrich (Merck)) |
| Enzyme 3 | Commercial | Commercial Esterase from porcine liver (PLE) (E3019, Sigma-Aldrich (Merck)) |
| Enzyme 4(1) | Commercial | Commercial lipase from *Aspergillus oryzae* (L0777, Sigma-Aldrich (Merck)) |
| Enzyme 4(2) | Commercial | Commercial lipase Novozym® 51032 from *Aspergillus oryzae* |
| Enzyme 5 | Commercial | Lipase from *Thermomyces lanuginosus* (commercial lipase Lipozyme® TL 100L) |
| Enzyme 6 | Commercial | Serine endopeptidase (commercial Savinase® 16L) |
| Enzyme 7 | Commercial | Protease from *Bacillus licheniformis* (commercial protease from *B. licheniformis* -P4860, Sigma-Aldrich (Merck)) |
| Enzyme 8 | In-house | Carboxylesterase/lipase<br>The amino acid sequence of which is SEQ ID NO: 4 |
| Enzyme 9 | In-house | Putative lactonizing lipase<br>The amino acid sequence of which is SEQ ID NO: 5 |

(continued)

| Enzyme as previously defined | Origin | Details |
|---|---|---|
| Enzyme 10 | In-house | Triglyceride lipase<br>The amino acid sequence of which is SEQ ID NO: 6 |
| Enzyme 11 | In-house | Hydrolase of alpha/beta superfamily<br>The amino acid sequence of which is SEQ ID NO: 7 |

Conclusions: Concerning the screening of the commercial enzymes for their capacity to degrade Substrate A and Substrate B, the tests have shown that all the commercial enzymes (enzymes 1 to 7) have degrading activity towards Substrate A and Substrate B (Figures 1 and 2).

Concerning the screening of the in-house enzymes for their capacity to degrade Substrate A and Substrate B, the tests have shown that all the in-house enzymes (enzymes 8 to 11) have degrading activity towards Substrate A and Substrate B (Figures 3 and 4).

***Cloning, over-expression and purification of 'in-house' enzymes of the invention***

Primers were chosen, genes were cloned and protein overexpressed in *E. coli* as previously described (Vergne-Vaxelaire et al., Adv. Synth. Catal. 2013, 355, 1763 - 1779). Each expression plasmid was transformed into *E. coli* (for example *E. coli* BL21-CodonPlus (DE3)-RIPL). Cell culture, isopropyl b-D-thiogalactopyranoside (IPTG) induction of protein production and cell lysis were conducted as previously published (C. Guérard-Hélaine et al. ChemCatChem 2015, 7, 1871-1879).

The enzymes were purified by loading the cell-free extract onto a Ni-NTA column (QIAGEN) according to the manufacturer's instructions. The elution buffer was 50 mM phosphate (pH 7.5), 50 mM NaCl, 250 mM imidazole and 10% glycerol and the desalting buffer was 50 mM phosphate (pH 7.5), 50 mM NaCl and 10% glycerol

The following Example refers to the 11 enzymes of the invention and listed above in Table 1, which have been identified by the BTB high-throughput assay.

**Degrading activity of the enzymes of the invention towards MR-7 and MR-8 polymer cubes**

The enzymes that were selected with the BTB assay on Substrate A and Substrate B (commercial, crude lysates and purified proteins) were tested for their capacity to degrade MR-7 and MR-8 polymer cubes (Hou *et al.,* 2019). Several experiments were conducted in which a cube of MR-7 or MR-8 polymer was mixed with one of the selected enzymes in a solution of sodium phosphate buffer 50 mM pH 7. Two enzyme concentrations were tested, either 200 or 2000 mU. The reaction mixture was incubated at 50°C, under constant agitation (150 rpm). Every 7 days, the reaction mixture was removed, and fresh buffer and enzymes were added. The reaction was stopped after 30 days.

To degrading activity of each enzyme was determined by calculating the weight loss of the polymer cube at the end of the reaction. The weight loss was calculated with the following formula:

$$\%\ \text{Degradation}\ (\%\ \text{weight loss}) = \frac{\text{Weight of polymer cube before reaction} - \text{Weight of polymer cube after reaction}}{\text{Weight of polymer cube before reaction}} \times 100$$

The most important weight loss and thus the most important polymer degradation have been obtained with enzyme 4(2) and enzyme 9 for MR-7 polymer and with enzyme 9 for MR-8 polymer.

**REFERENCES**

S. Tsushima, Y. Matsushita, Technical Report on the PCR-DGGE Analysis of Bacterial and Fungal Soil Communities, National Institute for Agro-Environmental Sciences, Tsukuba (2010).

A. Amobonye, P. Bhagwat, S. Singh, S. Pillai, Plastic biodegradation: frontline microbes and their enzymes, Sci. Total Environ., 759 (2021), p. 143536.

A.K. Urbanek, A.M. Mirończuk, A. Garcia-Martin, A. Saborido, I. Mata, M. Arroyo, Biochemical properties and biotechnological applications of microbial enzymes involved in the degradation of polyester-type plastics, BBA -

Proteins and Proteomics, 1868 (2020), p. 140315.

K. Mukai, Y. Doi, Y. Sema, K. Tomita, Substrate specificities in hydrolysis of polyhydroxyalkanoates by microbial esterases, Biotechnol. Lett., 15 (1993), pp. 601-604.

Toru Shimada and Takeshi Hasegawa, Determination of equilibrium structures of bromothymol blue revealed by using quantum chemistry with an aid of multivariate analysis of electronic absorption spectra, Spectrochimica Acta Part A: Mol. Biomol. Spectr., 185, 2017, 104-110.

Nazim Ocal, Aurélie Lagarde, Mélanie L'enfant, Franck Charmantray, and Laurence Hecquet, High-Throughput Solid-Phase Assay for Substrate Profiling and Directed Evolution of Transketolase, ChemBioChem, 2021, 22, 1-8.

Zhipeng Hou, Wei Zhang, Jing Guob, Zhangpei Chena, Jianshe Hua, Liqun Yang, The in vitro enzymatic degradation of poly(trimethylene carbonate-co-2, 2'-dimethyltrimethylene carbonate), Eur. Polym. J., 2019, 112, 51-59.

Vergne-Vaxelaire et al., Nitrilase Activity Screening on Structurally Diverse Substrates: Providing Biocatalytic Tools for Organic Synthesis, Adv. Synth. Catal. 2013, 355, 1763-1779.

Christine Guérard-Hélaine et al., Genome Mining for Innovative Biocatalysts: New Dihydroxyacetone Aldolases for the Chemist's Toolbox, ChemCatChem, 2015, 7, 1871-1879.

## Claims

**1.** A method for the enzymatic degradation of a polythiourethane (PTUR), comprising contacting said molecule with at least one enzyme capable of degrading said PTUR and selected from the group consisting of:

- enzyme 1, the amino acid sequence of which presents at least 60% identity with the following amino acid sequence:

MRVSLRSITSLLAAATAAVLAAPATETLDRRAALPNPYDDPFYTTPSNIGTFA
KGQVIQSRKVPTDIGNANNAASFQLQYRTTNTQNEAVADVATVWIPAKPASPPKIFS
YQVYEDATALDCAPSYSYLTGLDQPNKVTAVLDTPIIIGWALQQGYYVVSSDHEGFK
AAFIAGYEEGMAILDGIRALKNYQNLPSDSKVALEGYSGGAHATVWATSLADSYAPE
LNIVGASHGGTPVSAKDTFTFLNGGPFAGFALAGVSGLSLAHPDMESFIEARLNAKG
QQTLKQIRGRGFCLPQVVLTYPFLNVFSLVNDTNLLNEAPIAGILKQETVVQAEASYT
VSVPKFPRFIWHAIPDEIVPYQPAATYVKEQCAKGANINFSPYPIAEHLTAEIFGLVPS
LWFIKQAFDGTTPKVICGTPIPAIAGITTPSADQVLGSDLANQLRSLNGKQSAFGKPF
GPITPP (SEQ ID NO : 1 in the annexed sequence listing),

- enzyme 2, the amino acid sequence of which presents at least 60% identity with the following amino acid sequence:

SEVCFPRLGCFSDDAPWAGIVQRPLKILPWSPKDVDTRFLLYTNQNQNNYQ ELVADPSTITNSNFRMDRKTRFIIHGFIDKGEEDWLSNICKNLFKVESVNCICVDWKG GSRTGYTQASQNIRIVGAEVAYFVEVLKSSLGYSPSNVHVIGHSLGSHAAGEAGRR TNGTIERITGLDPAEPCFQGTPELVRLDPSDAKFVDVIHTDAAPIIPNLGFGMSQTVG HLDFFPNGGKQMPGCQKNILSQIVDIDGIWEGTRDFVACNHLRSYKYYADSILNPDG FAGFPCDSYNVFTANKCFPCPSEGCPQMGHYADRFPGKTNGVSQVFYLNTGDAS NFARWRYKVSVTLSGKKVTGHILVSLFGNEGNSRQYEIYKGTLQPDNTHSDEFDSD VEVGDLQKVKFIWYNNNVINPTLPRVGASKITVERNDGKVYDFCSQETVREEVLLTL NPC (SEQ ID NO: 2 in the annexed sequence listing),

- enzyme 3, the amino acid sequence of which presents at least 60% identity with the following amino acid sequence:

MWLLPLVLTSLASSATWAGQPASPPVVDTAQGRVLGKYVSLEGLAQPVAVF LGVPFAKPPLGSLRFAPPQPAEPWSFVKNTTSYPPMCCQDPVVEQMTSDLFTNGK ERLTLEFSEDCLYLNIYTPADLTKRGRLPVMVWIHGGGLVLGGAPMYDGVVLAAHE NVVVVAIQYRLGIWGFFSTGDEHSRGNWGHLDQVAALHWVQENIANFGGDPGSVT

IFGESAGGESVSVLVLSPLAKNLFHAISESGVALTVALVRKDMKAAAKQIAVLAGCKT TTSAVFVHCLRQKSEDELLDLTLKMKFLTLDFHGDQRESHPFLPTVVDGVLLPKMPE EILAEKDFNTVPYIVGINKQEFGWLLPTMMGFPLSEGKLDQKTATSLLWKSYPIANIP EELTPVATDKYLGGTDDPVKKKDLFLDLMGDVVFGVPSVTVARQHRDAGAPTYMY EFQYRPSFSSDKKPKTVIGDHGDEIFSVFGFPLLKGDAPEEEVSLSKTVMKFWANFA RSGNPNGEGLPHWPMYDQEEGYLQIGVNTQAAKRLKGEEVAFWNDLLSKEAAKK PPKIKHAEL (SEQ ID NO: 3 in the annexed sequence listing),

- enzyme 4, corresponding to a lipase from *Aspergillus oryzae,*
- enzyme 5 corresponding to a lipase from *Thermomyces lanuginosus,*
- enzyme 6 corresponding to a serine endopeptidase,
- enzyme 7, corresponding to a protease from *Bacillus licheniformis,*
- enzyme 8, the amino acid sequence of which presents at least 60% identity with the following amino acid sequence:

MTELTVQTRCGALKGTAGHGVRTWKSIPYAKPPVGELRFKAPEPPVPWDGV KNADSFGPVCPQPADLLSMSFSGDVPPQSEDCLYLNVFAPDSEGGKRPVMVWIHG GAFFLGAGSEPTYDASALAADGDVIVVTLNYRLGPFGFLHLFSIDDTYPGNIGMLDQI AALRWVKDNISAFGGDPDNVTVFGESAGGMSIASLMAMPDAKGLFHKAILESGASQ TMTADVAKEITTAFIQEAGTDQLQELSVNDILKTADKLRNTIDQSIFHLLFQPAIDPATL PAEPAKAIADGAAEGIPMIIGTNRDEAYLFFTPDTDIHSEKKQQEYLLYHLGENSAKL AADLYPHSLTGQIDMMTDLKFWRPAVAFAQEQSQYAPVWMYRFDWHGETPPFHK AVHALELPFVFGNFDSLKKTLKEPLSEDVKQLSKLIQSAWIAFAKTGKPDTDQLHWP QYETGSRETIIFNTSVSTESDPDSAKRRILFQA (SEQ ID NO: 4 in the annexed sequence listing),

- enzyme 9, the amino acid sequence of which presents at least 60% identity with the following amino acid sequence:

MSASSLKFPIVLVHGLLGFDKIGGIYPYFYGIKEALEKAGAKVYIATLSALNSNE LRGEQLLEFVRKVQAETGAAKVNLIGHSQGPLACRYVAATHPELIASVTSVNGVNH GSEVADLVRLALTPGRLPESIANAAMSAFGQLLSALAGSPRLPQSGIEALEALTSEG VAAFNKKYPQGLPAEWGGEGKELVNGVYYYSWSGIIDYNPLHQGANNLDPLHVAM LAFSILFTNERFQNDGLVGRYSSHLGKVIGSDYSMDHVDAINQLAGVVANNTDPVQL FVEHVARLKSKGL (SEQ ID NO: 5 in the annexed sequence listing),

- enzyme 10, the amino acid sequence of which presents at least 60% identity with the following amino acid sequence:

MEHGNRRGPGRACAHQDTVTLKAGTRRSTSAIARTAAGTAAAIAAAIAMAW PASATASSDYAKTRYPIVLVHGLTGAAKMGGVIDYWYGIPEVLRANGATVYVSTVPS FNSDEERALALQAYVRAVKLETGADKVNLIGHSQGGPTSRMLAAMSPQDVASVTTI GSPHRGSQLADVVLDVINGISSIPLAGPALVNVIQGVTNLLGWFNGLFNGQALNQDA IASARTLTSQGATEQNARLNATLAPGSQSALGPDCDTPGAVAEQRQGRDASGKLV TYTQAAYSWTGRGGPINLLRSNLLDPSTAAMAAAAGVMGMKGAGPNDGMVSVCS AKWGRVLSTDYYWNHLDEINQMLGMYQDVDPRTVILNHANRLRNDQL (SEQ ID NO: 6 in the annexed sequence listing),

- enzyme 11, the amino acid sequence of which presents at least 60% identity with the following amino acid sequence:

MKKLVALLIVGLFVSISLTGLGLAGGTAPYKCDTRYPVVLAHGMGASTKILGIV DYWYGIEDALKAEGASVYFTSVNAMGSTVDKAADFKQQFMEILAVTGAPKANIIGHS HGTLYTRYAISNLGLAPYVASYTSLAGPHRGSAVASLIMYDLPDWLLAAGGDVLNFV YTFIFGDTNPDSLQNALDLCPDYMVNTFNPNTPNIPGIYYQSWAAKAKTSCPSVILEP TWLIMLIEEGANDGLVSVESAKWGNFRGVEDAAWYSAGCDHLNIVGQLFGVTPGF DAPQFFVDIVEDLKGRGY (SEQ ID NO: 7 in the annexed sequence listing).

2. The method for the enzymatic degradation of a PTUR according to claim 1, wherein said enzyme 1 is a lipase A from *Candida antarctica.*

3. The method for the enzymatic degradation of a PTUR according to claim 1 or 2, wherein said enzyme 1 is the commercial lipase Novocor® AD-L.

4. The method for the enzymatic degradation of a PTUR according to any one of claims 1 to 3, wherein said enzyme 2 is a porcine pancreatic lipase, and preferably a commercial porcine pancreatic lipase.

5. The method for the enzymatic degradation of a PTUR according to any one of claims 1 to 4, wherein said enzyme 3 is an esterase from porcine liver and, preferably, a commercial esterase from porcine liver.

6. The method for the enzymatic degradation of a PTUR according to any one of claims 1 to 5, wherein said enzyme 4 is a commercial lipase from *Aspergillus oryzae.*

7. The method for the enzymatic degradation of a PTUR according to any one of claims 1 to 6, wherein said enzyme 4 is the commercial lipase Novozym® 51032 from *Aspergillus oryzae.*

8. The method for the enzymatic degradation of a PTUR according to any one of claims 1 to 7, wherein said enzyme 5 is the commercial lipase Lipozyme® TL 100L.

9. The method for the enzymatic degradation of a PTUR according to any one of claims 1 to 8, wherein said enzyme 6 is the commercial Savinase® 16L.

10. The method for the enzymatic degradation of a PTUR according to any one of claims 1 to 9, wherein said enzyme 7 is a commercial protease from *B. licheniformis.*

11. The method for the enzymatic degradation of a PTUR according to any one of claims 1 to 10, wherein said at least one enzyme is used in the form of a pure protein, a crude lysate or immobilized on a support.

12. The method for the enzymatic degradation of a PTUR according to any one of claims 1 to 11, wherein the polythiourethane is either MR-7 or MR-8.

13. Use of the method according to claim 12, for producing fragments, oligomers, monomers or repeating units derivatives from a polythiourethane that can be recovered and re-cycled for obtaining said polythiourethane.

14. A kit for the enzymatic degradation of a PTUR, comprising at least one enzyme as defined in any one of claims 1 to 11.

15. The kit of claim 14, further comprising at least one reagent for the degradation reaction.

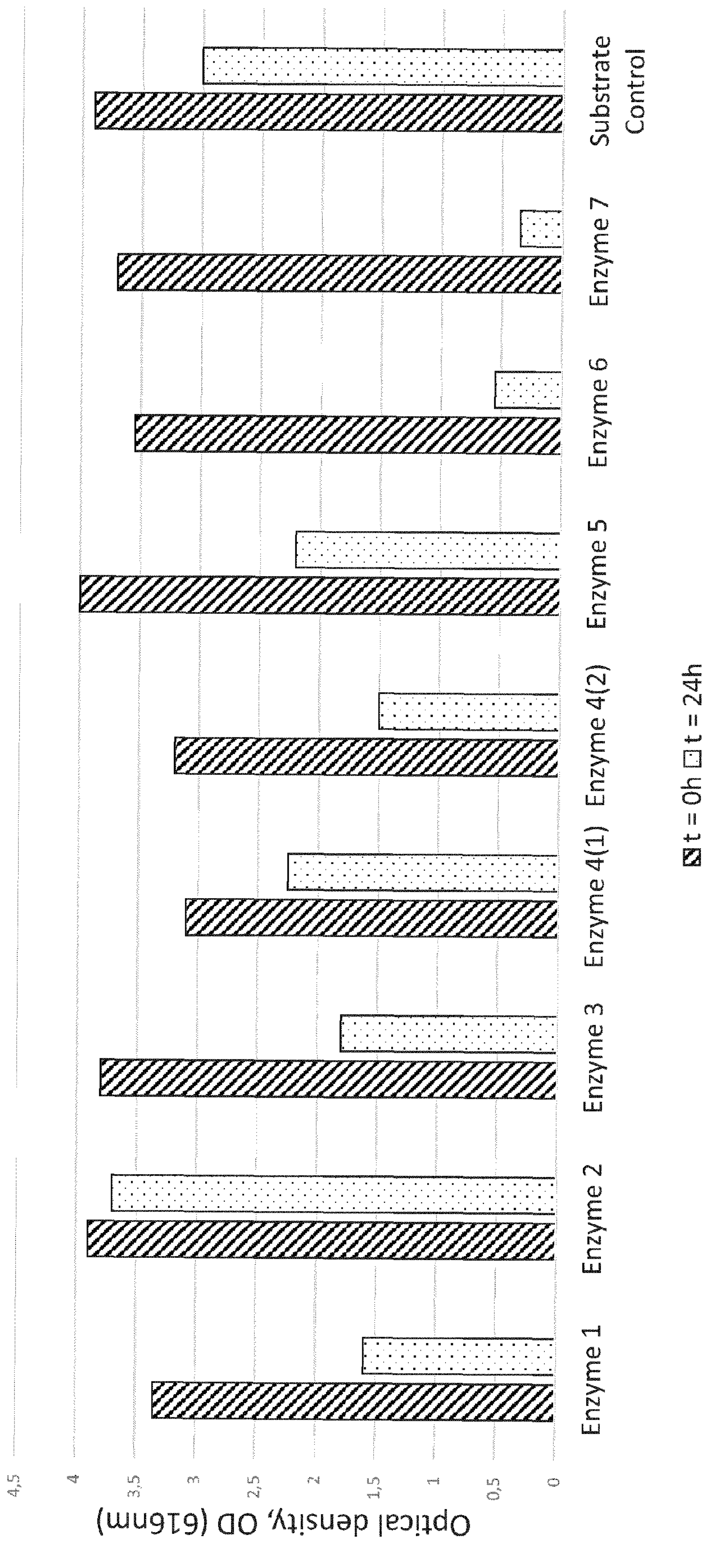
Optical density, OD (616nm)
0
0,5
1
1,5
2
2,5
3
3,5
4
4,5
Enzyme 1
Enzyme 2
Enzyme 3
Enzyme 4(1)
Enzyme 4(2)
Enzyme 5
Enzyme 6
Enzyme 7
Substrate Control
t = 0h
t = 24h

Figure 1

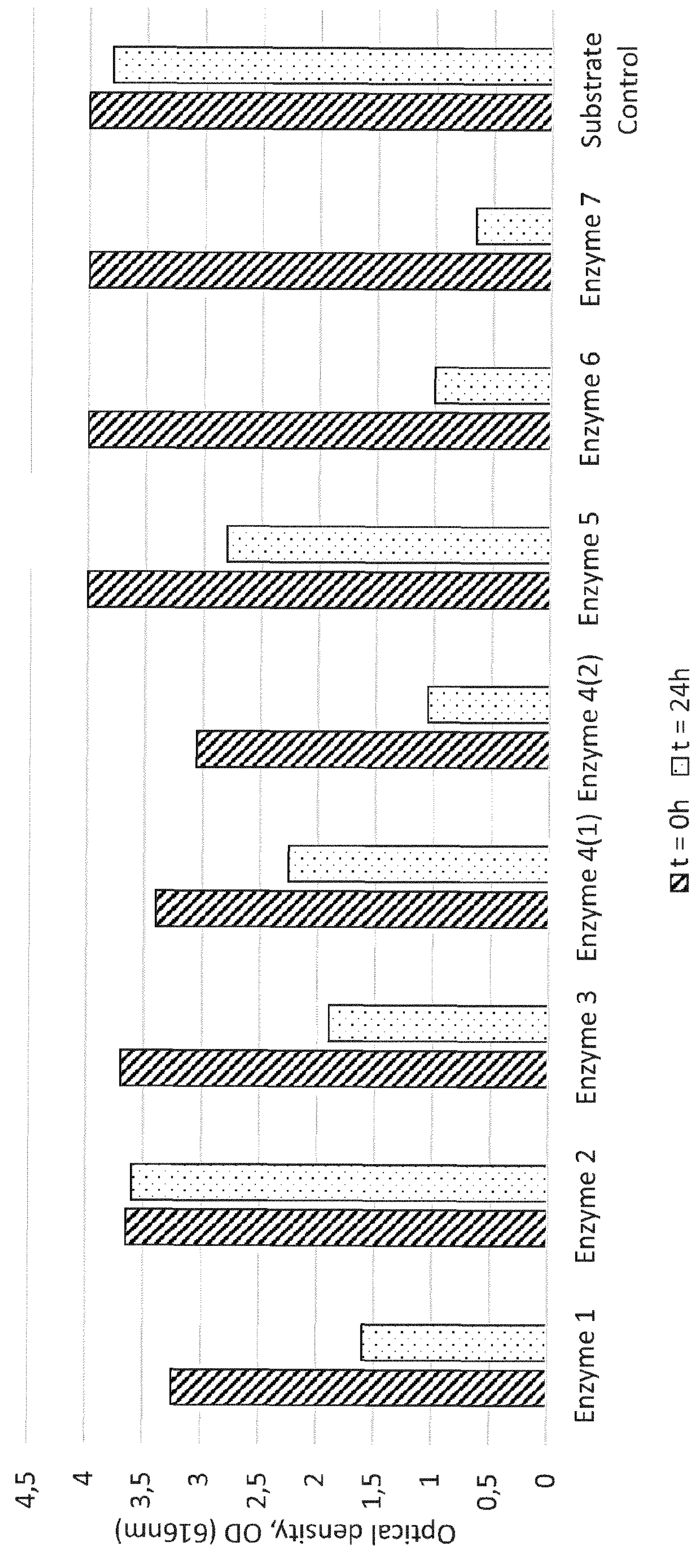
Optical density, OD (616nm)
4,5
4
3,5
3
2,5
2
1,5
1
0,5
0
Enzyme 1
Enzyme 2
Enzyme 3
Enzyme 4(1)
Enzyme 4(2)
Enzyme 5
Enzyme 6
Enzyme 7
Substrate Control
t = 0h
t = 24h

Figure 2

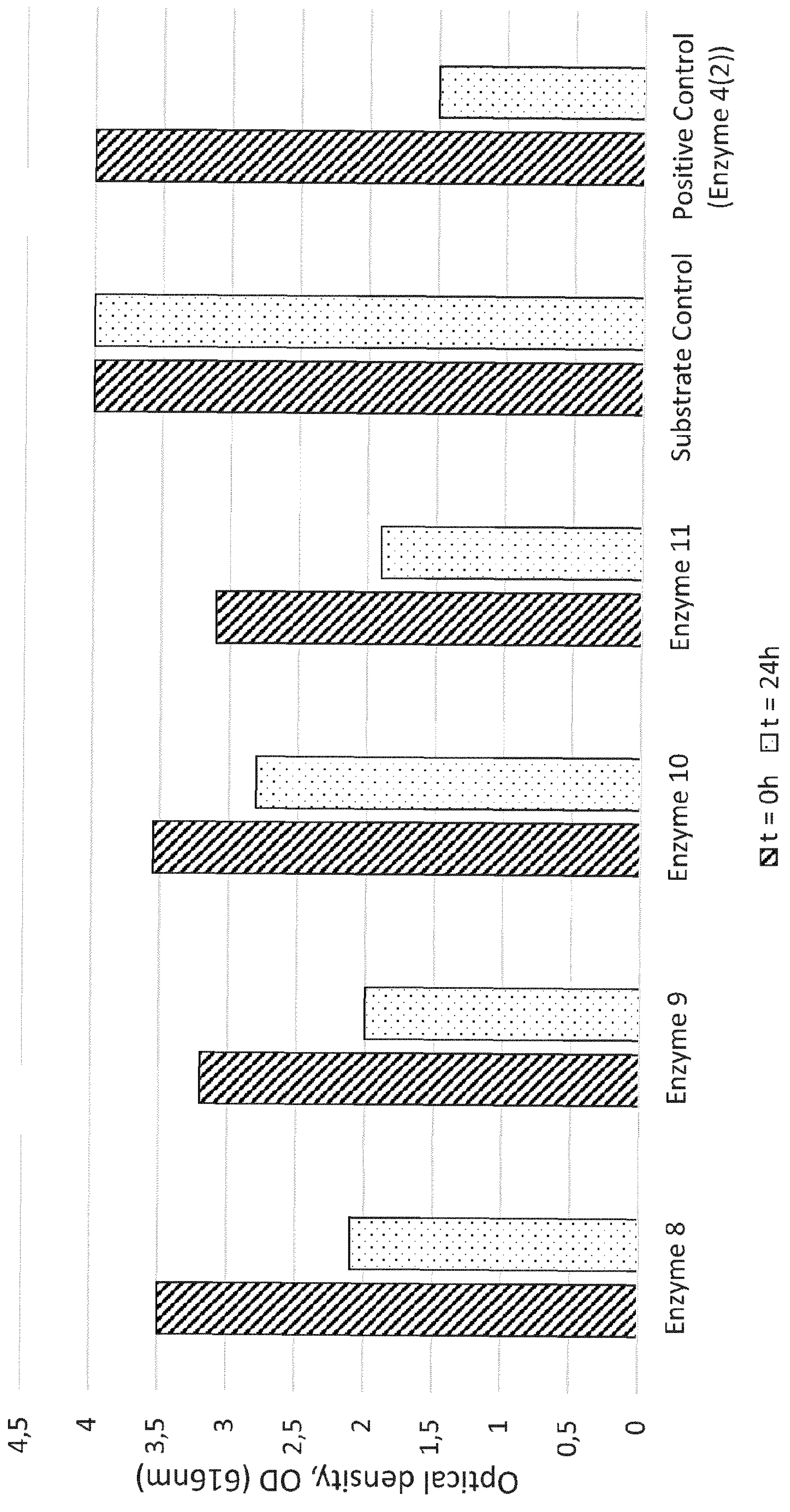
Optical density, OD (616nm)
4,5
4
3,5
3
2,5
2
1,5
1
0,5
0
Enzyme 8
Enzyme 9
Enzyme 10
Enzyme 11
Substrate Control
Positive Control (Enzyme 4(2))
t = 0h
t = 24h

Figure 3

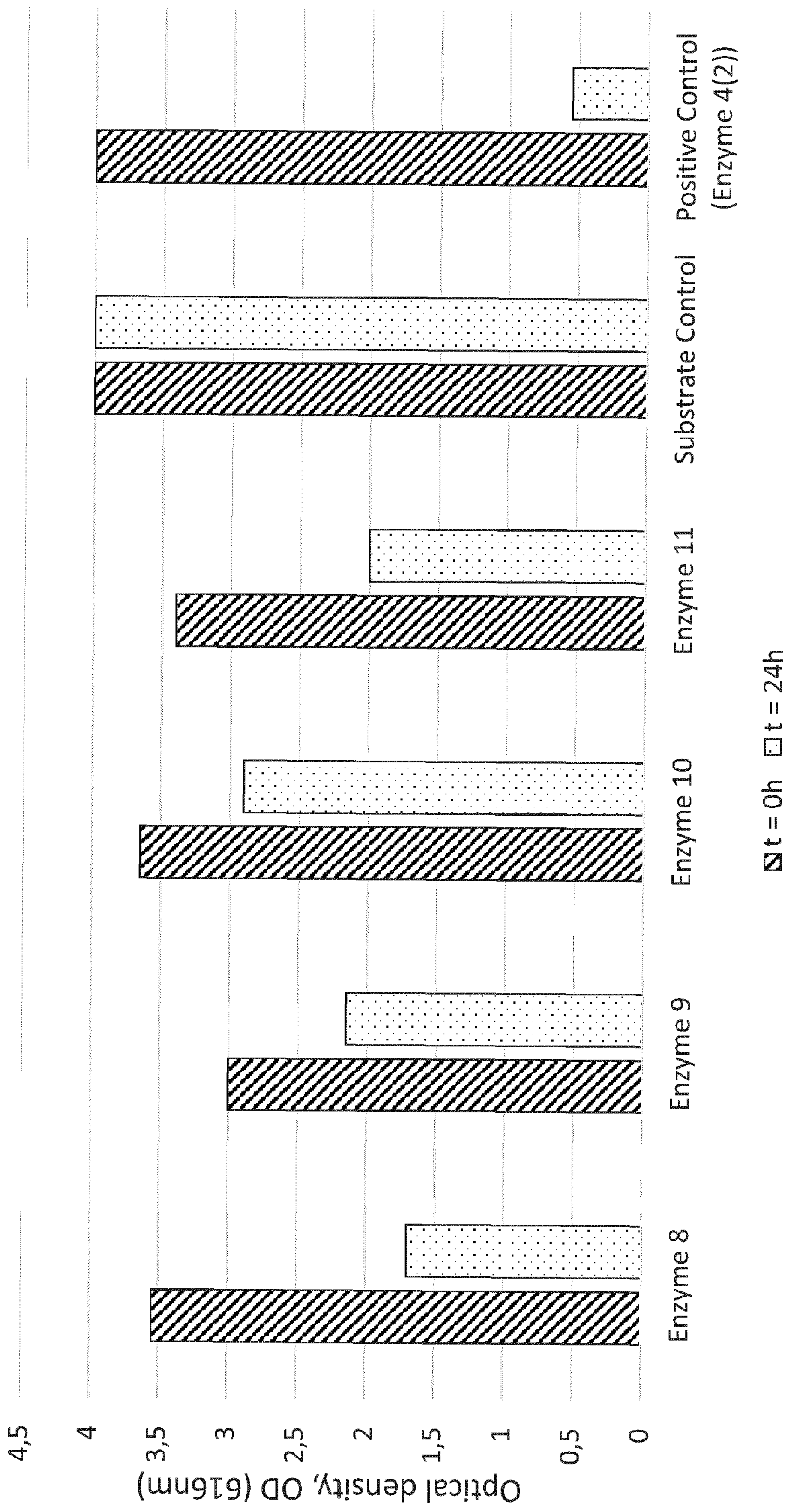

Optical density, OD (616nm)
0
0,5
1
1,5
2
2,5
3
3,5
4
4,5
Enzyme 8
Enzyme 9
Enzyme 10
Enzyme 11
Substrate Control
Positive Control (Enzyme 4(2))
t = 0h
t = 24h


Figure 4

Europäisches Patentamt
European Patent Office
Office européen des brevets

# EUROPEAN SEARCH REPORT

Application Number
EP 23 30 6475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FERRIS CRISTINA ET AL: "Dithiothreitol-based polyurethanes. Synthesis and degradation studies", POLYMER DEGRADATION AND STABILITY, vol. 95, no. 9, 1 September 2010 (2010-09-01), pages 1480-1487, XP093137659, GB ISSN: 0141-3910, DOI: 10.1016/j.polymdegradstab.2010.06.021 * abstract * * page 1481, left-hand column, paragraph 1-2 * * Scheme 1 * * page 1482, right-hand column, paragraph 6 - page 1485, right-hand column, last paragraph; figures 3,4; table 1 * ----- | 1,11,14 | INV. G08G18/3855 C12P7/13 C12P7/62 C12N9/18 C12N9/50 |
| A | HUANG SIJIA ET AL: "Chemical recycling of poly(thiourethane) thermosets enabled by dynamic thiourethane bonds", POLYMER CHEMISTRY, vol. 11, no. 43, 1 January 2020 (2020-01-01), pages 6879-6883, XP093137769, Cambridge ISSN: 1759-9954, DOI: 10.1039/D0PY01050B Retrieved from the Internet: URL:http://pubs.rsc.org/en/content/articlepdf/2020/PY/D0PY01050B> * abstract * * page 6680, right-hand column, paragraph 2 - page 6682, right-hand column, paragraph 1; figures 2-3 * ----- | 1-4,6-8, 11-15 | TECHNICAL FIELDS SEARCHED (IPC): C09J C08G C12P C12N |
| | -/-- | | |

~~The present search report has been drawn up for all claims~~

1

| Place of search | Date of completion of the search | Examiner |
|---|---|---|
| The Hague | 7 March 2024 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 30 6475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GAMARDELLA FRANCESCO ET AL: "Recyclable Organocatalyzed Poly(Thiourethane) Covalent Adaptable Networks", POLYMERS, vol. 12, no. 12, 4 December 2020 (2020-12-04), page 2913, XP093138289, CH ISSN: 2073-4360, DOI: 10.3390/polym12122913 * abstract * * page 13, paragraph 2 - page 15, paragraph 1; figures 8-10; table 5 * ----- | 1-4,6-8, 11-15 | |
| A | MAESTRI CLOTILDE ET AL: "Fungal Biodegradation of Polyurethanes", JOURNAL OF FUNGI, vol. 9, no. 7, 19 July 2023 (2023-07-19), page 760, XP093137780, ISSN: 2309-608X, DOI: 10.3390/jof9070760 ----- | 1-4,6-8, 11-15 | TECHNICAL FIELDS SEARCHED (IPC) |
| A | LOREDO-TREVIÑO ARACELI ET AL: "Polyurethane as substrate for fungal strains", ADVANCES IN BIOSCIENCE AND BIOTECHNOLOGY, vol. 02, no. 02, 1 January 2011 (2011-01-01), pages 52-58, XP093137775, ISSN: 2156-8456, DOI: 10.4236/abb.2011.22009 Retrieved from the Internet: URL:http://www.scirp.org/journal/doi.aspx?DOI=10.4236/abb.2011.22009> ----- | 1-4,6-8, 11-15 | |

1 ~~The present search report has been drawn up for all claims~~

| Place of search | Date of completion of the search | Examiner |
|---|---|---|
| The Hague | 7 March 2024 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 23 30 6475

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  2-4, 6-8(completely); 1, 11-15(partially)

- [ ] The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 23 30 6475

The Search Division considers that the present European patent application does not comply with the requirements of unity of invention and relates to several inventions or groups of inventions, namely:

1. claims: 2-4, 6-8(completely); 1, 11-15(partially)

A method for the enzymatic degradation of a polythiourethane (PTUR), comprising contacting said molecule with at least one enzyme capable of degrading said PTUR and selected from the group consisting of lipases represented by amino acid sequences which present 60% identity with SEQ ID NOs. 1-2, lipases from Aspergillus oryzae, lipase from Thermomyces lanuginosus, a carboxylesterase lipase represented by SEQ ID NO 4, a putative lactonizing lipase with SEQ ID NO 5, and a putative tryglyceride lipase with SEQ ID NO 6.

---

2. claims: 5(completely); 1, 11-15(partially)

A method for the enzymatic degradation of a polythiourethane (PTUR), comprising contacting said molecule with at least one enzyme capable of degrading said PTUR and selected from the group consisting of an enzyme represented by amino acid sequences which present 60% identity with SEQ ID NO. 3.

---

3. claims: 9(completely); 1, 11-15(partially)

A method for the enzymatic degradation of a polythiourethane (PTUR), comprising contacting said molecule with at least one enzyme capable of degrading said PTUR and corresponding to a serine endopeptidase.

---

4. claims: 10(completely); 1, 11-15(partially)

A method for the enzymatic degradation of a polythiourethane (PTUR), comprising contacting said molecule with at least one enzyme capable of degrading said PTUR and corresponding to a from a protease from Bacillus licheniformis.

---

5. claims: 1, 11-15(all partially)

A method for the enzymatic degradation of a polythiourethane (PTUR), comprising contacting said molecule with at least one enzyme capable of degrading said PTUR and selected from the group consisting of lipases represented by amino acid sequences which present 60% identity with SEQ ID NO. 7.

---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4689387 A
- US 4775733 A
- US 5059673 A
- US 5087758 A
- US 5191055 A


### Non-patent literature cited in the description


- *CHEMICAL ABSTRACTS*, 9016-18-6
- **VERGNE-VAXELAIRE et al.** *Adv. Synth. Catal.*, 2013, vol. 355, 1763-1779
- **C. GUÉRARD-HÉLAINE et al.** *ChemCatChem*, 2015, vol. 7, 1871-1879
- **S. TSUSHIMA** ; **Y. MATSUSHITA**. Technical Report on the PCR-DGGE Analysis of Bacterial and Fungal Soil Communities. *National Institute for Agro-Environmental Sciences, Tsukuba*, 2010
- **A. AMOBONYE** ; **P. BHAGWAT** ; **S. SINGH** ; **S. PILLAI**. Plastic biodegradation: frontline microbes and their enzymes. *Sci. Total Environ*, 2021, vol. 759, 143536
- **A.K. URBANEK** ; **A.M. MIROŃCZUK** ; **A. GARCIA-MARTIN** ; **A. SABORIDO** ; **I. MATA** ; **M. ARROYO**. Biochemical properties and biotechnological applications of microbial enzymes involved in the degradation of polyester-type plastics. *BBA - Proteins and Proteomics*, 2020, vol. 1868, 140315
- **K. MUKAI** ; **Y. DOI** ; **Y. SEMA** ; **K. TOMITA**. Substrate specificities in hydrolysis of polyhydroxyalkanoates by microbial esterases. *Biotechnol. Lett.*, 1993, vol. 15, 601-604
- **TORU SHIMADA** ; **TAKESHI HASEGAWA**. Determination of equilibrium structures of bromothymol blue revealed by using quantum chemistry with an aid of multivariate analysis of electronic absorption spectra. *Spectrochimica Acta Part A: Mol. Biomol. Spectr.*, 2017, vol. 185, 104-110
- **NAZIM OCAL** ; **AURÉLIE LAGARDE** ; **MÉLANIE L'ENFANT** ; **FRANCK CHARMANTRAY** ; **LAURENCE HECQUET**. High-Throughput Solid-Phase Assay for Substrate Profiling and Directed Evolution of Transketolase. *ChemBioChem*, 2021, vol. 22, 1-8
- **ZHIPENG HOU** ; **WEI ZHANG** ; **JING GUOB** ; **ZHANGPEI CHENA** ; **JIANSHE HUA** ; **LIQUN YANG**. The in vitro enzymatic degradation of poly(trimethylene carbonate-co-2, 2'-dimethyltrimethylene carbonate). *Eur. Polym. J*, 2019, vol. 112, 51-59
- **VERGNE-VAXELAIRE et al.** Nitrilase Activity Screening on Structurally Diverse Substrates: Providing Biocatalytic Tools for Organic Synthesis. *Adv. Synth. Catal*, 2013, vol. 355, 1763-1779
- **CHRISTINE GUÉRARD-HÉLAINE et al.** Genome Mining for Innovative Biocatalysts: New Dihydroxyacetone Aldolases for the Chemist's Toolbox. *ChemCatChem*, 2015, vol. 7, 1871-1879